Europäisches Patentamt

European Patent Office

Office européen des brevets

(19)

(11) Veröffentlichungsnummer: **0 079 466**
**A2**

(12) **EUROPÄISCHE PATENTANMELDUNG**

(21) Anmeldenummer: **82109566.8**

(22) Anmeldetag: **15.10.82**

(51) Int. Cl.³: **C 07 G 7/00**
**B 01 D 15/08, G 01 N 33/54**
**A 61 K 39/44**

(30) Priorität: **12.11.81 DE 3145007**

(43) Veröffentlichungstag der Anmeldung:
**25.05.83 Patentblatt 83/21**

(84) Benannte Vertragsstaaten:
**AT BE CH DE FR GB IT LI LU NL SE**

(71) Anmelder: **Max-Planck-Gesellschaft zur Förderung der Wissenschaften e.V.**
**Bunsenstrasse 10**
**D-3400 Göttingen(DE)**

(72) Erfinder: **Maelicke, Alfred, Prof. Dr.**
**Augsburger Strasse 69**
**D-4757 Holzwickede(DE)**

(74) Vertreter: **Weickmann, Heinrich, Dipl.-Ing. et al,**
**Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22**
**D-8000 München 86(DE)**

(54) **Immobilisierte biologisch aktive Substanzen und ihre Verwendung.**

(57) Die Erfindung betrifft immobilisierte biologisch aktive Substanzen aus der Gruppe Acetylcholinrezeptoren und monoclonale Antikörper. Das Trägermaterial ist z.B. ein partiell hydrolysiertes Polyamid. Die erfindungsgemäßen immobilisierten biologisch aktiven Substanzen eignen sich zur Verwendung in der Affinitätschromatographie, insbesondere der Immunosorption, und für die Diagnose und Therapie von Autoimmunkrankheiten, wie z.B. der Myasthenia gravis.

EP 0 079 466 A2

**0079466**

B e s c h r e i b u n g

Die Erfindung betrifft immobilisierte biologisch aktive Substanzen und ihre Verwendung in der Affinitätschromatographie, insbesondere der Immunosorption, und für die Diagnose und/oder Therapie von Autoimmunkrankheiten, insbesondere von Myasthenia gravis.

An einem festen, unlöslichen Träger fixierte biologisch aktive Substanzen, wie z. B. Enzyme, Cofaktoren, Antigene oder Antikörper, erlangen in vielen Anwendungsbereichen der Forschung, Industrie und Medizin zunehmende Bedeutung. Insbesondere organische Polymere in der verschiedensten Gestalt und mit einer zur chemischen oder physikalischen Bindung des biologisch aktiven Materials behandelten Oberfläche haben sich als Trägersubstanzen bewährt (vgl. z. B. DE-OS 24 37 870, DE-OS 24 38 436, US-PS 3 970 597, Inman und Hornby, Biochem. J. 129, 1972, 255).

Gegenstand der vorliegenden Erfindung sind immobilisierte biologisch aktive Substanzen aus der Gruppe Acetylcholinrezeptoren und monoclonale Antikörper, und insbesondere solche, die an einen vorzugsweise schlauchförmigen Polyamidträger an dessen innere Oberfläche gebunden sind.

Als Trägermaterialien können alle bekannten anorganischen und insbesondere organischen Trägermaterialien verwendet werden, die sich zur ausreichenden Fixierung der erfindungsgemäßen biologisch aktiven Substanzen unter weitgehender Erhaltung ihrer Aktivität, gegebenenfalls nach einer physikalischen oder chemischen Oberflächbehandlung, eignen. Besonders geeignet sind dabei Träger auf Basis von natürlichen oder synthetischen Kunststoffen, Biopolymeren oder anderen organischen Substanzen, bei denen aktive Gruppen vorhanden sind oder leicht erzeugt werden können, und die möglichst hydrophile Gruppen oder Zentren enthalten, wie z. B. Polyamide, Cellulose, Cellulosederivate oder andere Polysaccharide, Polyacrylamid usw.

Die Form der Träger richtet sich insbesondere nach dem beabsichtigten Verwendungszweck; sie können z. B. in Form von Pulvern, Granulaten, Membranen, Netzen, Folien oder in anderer Gestalt vorliegen, und insbesondere als Schläuche, die sich für diagnostische und therapeutische Anwendungen als besonders geeignet erwiesen haben. An den schlauchförmigen Trägermaterialien, die auch natürliche oder synthetische Hohlfasersysteme sein können, befinden sich die biologisch aktiven Substanzen insbesondere an oder auch an der Innenseite.

Für bestimmte Anwendungen kann es dabei zweckmäßig sein, semipermeable Hohlfasern einzusetzen

Erfindungsgemäß werden als Trägermaterial vorzugsweise Polyamide, wie z. B. Nylon-6, Nylon-6,10 oder Nylon-66, eingesetzt, die zur Vergrößerung ihrer Bindungsfähigkeit gegenüber den biologisch aktiven Liganden "aktiviert" sind. Die Erhöhung der Bindungsfähigkeit kann nach an sich bekannten Methoden erfolgen, wie z. B. durch Hydrolyse (teilweise Verseifung der Amidbrücken) und gegebenenfalls nachfolgende Umsetzung mit einem Seitenketten bildenden Reagens, wie z. B. mit Aldehyd und Isonitril, oder durch O-Alkylierung mit Dimethylsulfat, Triäthyloxoniumtetrafluoroborat oder anderen Alkylierungsmitteln (vgl. z. B. DE-OS 24 37 870, US-PS 3 970 597, P.V. Sundaram et al., Clin. Chem. 24, 1978, 234).

Bei den als biologisch aktive Substanzen erfindungsgemäß eingesetzten Acetylcholinrezeptoren handelt es sich um Membranproteine, die z. B. aus Torpedo marmorata isoliert werden können (vgl. Rüchel et al., Eur. J. Biol. Chem. 119 (1981), 215 bis 223 ). Sie werden vorzugsweise in gereinigter Form (Reinigungsmethode nach Maelicke et al., Handbook of Physiology, The Nervous System I, 14, 1977, Seite 493-519; J. Biol. Chem. 252, 1977, 4811 bis 4830) verwendet, wobei auch Untereinheiten (subunits) eingesetzt werden können.

Erfindungsgemäß eingesetzte monoclonale Antikörper sind monospezifische Antikörper, wie z. B. Antiidiotyp-Antikörper, die gegen die im Blut von immunisierten Kaninchen auftretenden Antikörper erzeugt werden.

Die erfindungsgemäßen Systeme können in der Affinitätschromatographie, insbesondere der Immunosorption, verwendet werden. So wurde z. B. gefunden, daß sich auf Nylon als Trägersubstanz immobilisierte Acetylcholinrezeptoren oder deren Untereinheiten (subunits) sehr gut als Adsorbens in der Affinitätschromatographie zur Reinigung von rezeptorbindenden Proteinen, Neurotoxinen oder Antikörpern eignen. Dabei können Nachteile, wie insbesondere nicht-kovalente Adsorption an die Matrix, Instabilität und Veränderung der Bindungseigenschaften des Rezeptors, vermieden werden.

Im Hinblick auf ihre Verwendung besonders geeignete erfindungsgemäße Systeme sind solche, an denen die biologisch aktive Substanz an einen aktivierten Polyamid-(Nylon)- Schlauch als Träger gebunden ist. Der innere Durchmesser der Schläuche ist in weiten Grenzen variierbar; er beträgt z. B. o,1 cm.    Die Aktivierung erfolgt dabei vorzugsweise durch partielle Hydrolyse mit Salzsäure, wie z. B. mit 3,5-molarer Salzsäure bei 70°C während einer Inkubationszeit von 7 Minuten, und nachfolgendem mehrstündigem sorgfältigem Waschen mit kaltem Wasser oder Pufferlösung (Sundaram und Hornby, FEBS Lett. 10, 1970, 325 bis 327, Sundaram, Biochemical Applications of Immobilized Enzymes and Proteins, Plenum Press, New York 1977, 317 bis 340). Daran anschließend erfolgt die Bindung der biologisch aktiven Substanz an den aktivierten Träger vorzugsweise unter Verwendung von bifunktionellen Reagenzien, insbesondere mittels Glutaraldehyd oder in erster Linie mittels 1-Äthyl-3-(3-dimethylamino-propyl)-carbodiimid-hydrochlorid (EDAC), z.B. nach der Methode von Gordon et al., Ultrastruct. Res. 47, 1974, 285 bis 295.

Die Brauchbarkeit immobilisierter biologisch aktiver Substanzen hängt insbesondere von der Erhaltung ihrer biospezifischen Eigenschaften bei der Bindung an den Träger und nach mehrmaliger Verwendung in der Chromatographie ab. Es hat sich nun gezeigt, daß bei den erfindungsgemäß eingesetzten biologisch aktiven Substanzen diese Eigenschaften, insbesondere die toxinbindenden Eigenschaften der Acetylcholinrezeptoren, besonders weitgehend erhalten bleiben, wenn man die bevorzugt genannten Systeme, in denen die biologisch aktiven Substanzen unter Verwendung von Glutaraldehyd, und insbesondere von EDAC an den aktivierten Nylonschlauch gebunden sind, verwendet. Die biospezifischen Eigenschaften bleiben auch nach längerem Gebrauch und Lagerung des Systems erhalten. Die Acetylcholinrezeptor-Nylon-Affinitätsschläuche können z. B. im feuchten Zustand über Monate ohne wesentlichen Verlust ihrer biospezifischen Aktivität aufbewahrt werden.

Weitere Vorteile dieser Systeme sind ihre hohe Dichte an trägergebundener biologisch aktiver Substanz, ihre Stabilität und ihre gute und sterile Lagerfähigkeit und Wiederverwendbarkeit. Es wurde gefunden, daß diese Systeme sich auch besonders gut zur Affinitätschromatographie von solubilisierten Membranproteinen eignen, weil die Proteine, im Vergleich zu anderen Matrixsystemen, damit viel weniger denaturiert oder in ihren Eigenschaften modifiziert werden. Sie eignen sich auch gut für die Bestimmung von Antikörpertitern in Kulturbeständen, Seren und anderen Flüssigkeiten, und für Reinigungszwecke z. B. bei Allergien, Vergiftungen usw. Wegen der hohen Packungsdichte der an die Trägeroberfläche gebundenen biologisch aktiven Substanzen und des kleinen Volumens der Schläuche bewirken diese Systeme bei der Immunosorption einen hohen Anreicherungseffekt und ermöglichen die sehr empfindliche Bestimmung von Antikörpertitern.

Ein besonderer Vorteil der erfindungsgemäß immobilisierten biologisch aktiven Substanzen, insbesondere der bevorzugt genannten Systeme, in denen die biologisch aktiven Substanzen an aktivierte Nylonschläuche gekuppelt sind, besteht in ihrer überraschend guten Eignung zur Diagnose und/oder Therapie von Autoimmunkrankheiten, insbesondere von Myasthenia gravis.

Bei Myasthenia gravis handelt es sich um eine Autoimmunkrankheit. In Myasthenia gravis-Patienten tritt eine neuromuskuläre Blockade auf, die durch erniedrigte Muskelaktionspotentiale bei gleicher Neurostimulation charakterisiert ist. Diese Blockade kann durch Cholinesteraseblocker teilweise aufgehoben werden. Myasthenia gravis wird durch ein Störverhalten der Endplatten verursacht. Neben diesen direkten Effekten an der Endplatte sind für Myasthenie-Kranke Veränderungen der Thymusdrüse typisch. Diese ist bei zwei Drittel der Patienten typisch hervortretend und hat verhärtete Zentren, bei einem Zehntel der Patienten sind Tumoren der Thymusdrüse zu finden. In den meisten Fällen verbessert eine Thymektomie die Symptome der Krankheit wesentlich. Da die Thymusdrüse verantwortlich ist für die funktionelle Entwicklung der T-Lymphozyten und da die verhärteten Zentren geschwollener Thymusdrüsen gewöhnlich B-Lymphozyten enthalten, war schon seit langem eine Störung des Immunsystems als Ursache der Krankheit vermutet worden. Mitte der 60er Jahre konnte dann gezeigt werden, daß bei etwa der Hälfte von Myasthenie-Kranken Eiweißkörper im Serum gefunden werden, die mit Antigenkompo-

nenten von Muskelmembranen reagieren. Ein unmittelbarer Effekt dieser Globuline auf die cholinerge Reizung von Muskelzellen konnte jedoch nicht gezeigt werden. Mit Hilfe von gereinigten Acetylcholinrezeptor-Präparationen wurde dann gezeigt, daß Globuline in Serum von wenigstens 75 % aller Myasthenie-Kranken mit gereinigten Rezeptoren reagieren. Obwohl bisher kein direkter Effekt der Antirezeptor-Antikörper im Serum von Myasthenie-Kranken auf die neuromuskuläre Transmission gezeigt werden konnte, kann eine experimentelle neuromuskuläre Blockade bei Tieren durch Immunisierung mit gereinigtem Rezeptorprotein erzeugt werden. Experimentelle Myasthenie kann passiv durch Zellen der Lymphknoten und teilweise auch durch Serum übertragen werden. Sie entwickelt sich nicht in thymektomierten Tieren, so daß Myasthenia gravis eine T-Zellen-abhängige Krankheit ist.

Myasthenia gravis kann durch den direkten Nachweis von Antikörpern gegen Acetylcholinrezeptoren im Blut der Patienten diagnostiziert werden. Diese Diagnose ist aber nur in wenigen Krankenhäusern möglich, da Acetylcholinrezeptoren normalerweise sehr empfindliche Proteine sind, die nicht ohne weiteres verschickt und gehandhabt werden können. Außerdem ist diese Methode zur Frühdiagnose ungeeignet, da diese Techniken eine zu schlechte Erfassungsgrenze besitzen. Ein besonderes Problem ergibt sich noch dadurch, daß die Antirezeptor-Immuntiter in Serum von Myasthenie-Kranken keine eindeutige Parallele zur Schwere der Krankheit darstellen. Es muß daher vermutet werden, daß nur eine begrenzte spezifisch wirkende Population der gegen den Rezeptor gerichteten Antikörper pathogen wirkt. Die häufigste Therapiemethode ist noch immer Thymektomie, obwohl diese einen massiven Eingriff in die Regulation des körpereigenen Immunsystems bedeutet.

Mit den erfindungsgemäßen Systemen lassen sich die aufgezeigten Mängel und Nachteile der bisherigen Diagnose- und Therapiemethoden überwinden. Aufgrund ihrer hohen Stabilität und Lagerfähigkeit sind die erfindungsgemäßen Systeme jederzeit und überall einsetzbar. Sie eignen sich auch gut zur Frühdiagnose. Außerdem läßt sich mit ihnen auch die Schwere der Erkrankung innerhalb enger, tolerierbarer Fehlergrenzen bestimmen.

Gegenstand der Erfindung ist deshalb auch der Einsatz der erfindungsgemäßen immobilisierten biologisch aktiven Substanzen zur Verwendung für die Diagnose und/oder Therapie von Autoimmunkrankheiten, und insbesondere von Myasthenia gravis.

Zum Nachweis der therapeutischen Eignung wurde das Blut von Acetylcholinrezeptor-immunisierten und damit experimentell myasthenischen Kaninchen extrakorporal durch ein auf Nylonschläuchen immobilisiertes Acetylcholinrezeptor-System gemäß dem nachstehenden Beispiel geführt. Die typischen Symptome der Myasthenie können auf diese Weise über lange Zeiten unterdrückt werden. Damit kann auch ein eindeutiger Zusammenhang zwischen niedrigen Antikörpertitern gegen Acetylcholinrezeptoren und Zurückdrängung der Symptome der Myasthenie gezeigt werden.

In einem weiteren Versuch wurden gegen die im Blut von immunisierten Kaninchen auftretenden Antikörper Antiidiotyp-Antikörper erzeugt. Nach deren Selektionierung wurden die entsprechenden Original-Antikörper, die für die Symptome der Krankheit verantwortlich sind, spezifisch aus dem Serum herausgefiltert. Diese monoclonalen Antikörper wurden auf einem gemäß dem nachstehenden Beispiel aktivierten Nylonschlauch immobilisiert. Leitet man durch dieses System das Blut von experimentell myasthenischen Kaninchen, so können die typischen Symptome der Myasthenie über lange Zeit unterdrückt werden. Beide vorstehend beschriebenen Methoden sind für eine Anwendung beim Menschen geeignet.

Das nachfolgende Beispiel erläutert die Erfindung näher, ohne sie darauf zu beschränken.

B e i s p i e l

Ein Nylonschlauch mit einem Innendurchmesser von 0,1 cm wird mit 3,5-molarer Salzsäure bei 70°C 7 Minuten lang partiell hydrolysiert und danach mit einer Pufferlösung (pH = 6) mehrere Stunden gewaschen. Dann wird eine wäßrige Lösung von 1-Äthyl-3-(3-dimethylamino-propyl)-carbodiimidhydrochlorid (100 mMol, mit 10 mmolarer Salzsäure auf pH = 5 eingestellt) durch den Schlauch geleitet. Nach 2 Stunden wird der Schlauch 5 Minuten lang mit Wasser gewaschen und dann 24 Stunden lang eine kalte Acetat-Pufferlösung (oder Wasser mit gleichem pH-Wert), die den solubilisierten Acetylcholinrezeptor enthält, hindurchgeleitet. Daran anschließend wird der Schlauch mit 0,3 molarer wäßriger Natriumchlorid-Lösung und Wasser gewaschen und mit Pufferlösung gefüllt bei 4°C aufbewahrt. In einer zweckmäßigen Ausführungsform des Verfahrens wird der Nylonschlauch um einen Stab gewickelt und die Lösungen mit einer Geschwindigkeit von 0,2 ml/min hindurchgepumpt.

Die Menge an trägergebundenem Acetylcholinrezeptor wurde durch Gehaltsbestimmung der Rezeptorlösungen vor und nach der Perfusion bestimmt. Die effektive Rezeptorkonzentration am Träger wurde mittels $^{125}$Jod-markiertem Toxin bestimmt.

Die erhaltene "Kupplungseffizienz" (Mol x $10^{-12}$ Acetylcho-

linrezeptor/1 cm Länge des Nylonschlauchs) betrug 137.

Für $\alpha$-Neurotoxin wurde die Bindungskapazität (trapping capacity) des Systems bestimmt (unter Verwendung von $^{125}$Jod-markiertem $\alpha$-Neurotoxin). Sie betrug $36 \times 10^{-12}$ Mol/cm.

Zur Aufbewahrung werden die erhaltenen Nylonschläuche zweckmäßigerweise mit einer Proteaseinhibitoren enthaltenden Pufferlösung (z. B. 0,1 M Benzamidin, $10^{-6}$ M Trasylol, 1 mM Diisopropylfluorophosphat) gefüllt.

Immobilisierte biologisch aktive Substanzen und ihre
Verwendung

P a t e n t a n s p r ü c h e

1. Immobilisierte biologisch aktive Substanzen aus der Gruppe Acetylcholinrezeptoren und monoclonale Antikörper.

2. Immobilisierte biologisch aktive Substanzen nach Anspruch 1, d a d u r c h   g e k e n n z e i c h n e t , daß das Trägermaterial ein partiell hydrolysiertes Polyamid ist.

3. Immobilisierte biologisch aktive Substanzen nach Anspruch 1 oder 2, d a d u r c h   g e k e n n z e i c h - n e t , daß die biologisch aktiven Substanzen an das Trägermaterial mittels bifunktioneller Reagenzien, insbesondere mittels Glutaraldehyd oder 1-Äthyl-3-(3-dimethylamino-propyl)-carbodiimid-hydrochlorid fixiert sind.

4.    Immobilisierte biologisch aktive Substanzen nach einem der Ansprüche 1 bis 3, d a d u r c h   g e k e n n - z e i c h n e t , daß das Trägermaterial schlauchförmig ist und die biologisch aktiven Substanzen auf der Innenseite fixiert sind.

5.    Immobilisierte biologisch aktive Substanzen nach einem der Ansprüche 1 bis 4, d a d u r c h   g e k e n n - z e i c h n e t , daß die Trägermaterialien Kohlfasern sind.

6.    Immobilisierte biologisch aktive Substanzen nach einem der Ansprüche 1 bis 5 zur Verwendung in der Affinitätschromatographie, insbesondere Immunosorption.

7.    Immobilisierte biologisch aktive Substanzen nach einem der Ansprüche 1 bis 5 zur Verwendung für die Diagnose und/oder Therapie von Autoimmunkrankheiten.

8.    Immobilisierte biologisch aktive Substanzen nach einem der Ansprüche 1 bis 5 zur Verwendung für die Diagnose und/oder Therapie von Myasthenia gravis.